(19) Europäisches Patentamt
European Patent Office
Office européen des brevets

(11) Veröffentlichungsnummer: **0 082 822**
**B1**

# (12) EUROPÄISCHE PATENTSCHRIFT

(45) Veröffentlichungstag der Patentschrift: **17.09.86**

(51) Int. Cl.⁴: **C 07 D 491/04** // B41M5/12, (C07D491/04, 307:00, 221:00)

(21) Anmeldenummer: **82810549.4**

(22) Anmeldetag: **17.12.82**

## (54) Chromogene Dihydrofuropyridinone, Verfahren zu ihrer Herstellung und ihre Verwendung in druck- oder wärmeempfindlichen Aufzeichnungsmaterialien.

(30) Priorität: **23.12.81 CH 8250/81**
**23.12.81 CH 8251/81**

(43) Veröffentlichungstag der Anmeldung: **29.06.83 Patentblatt 83/26**

(45) Bekanntmachung des Hinweises auf die Patenterteilung: **17.09.86 Patentblatt 86/38**

(84) Benannte Vertragsstaaten: **BE CH DE FR GB IT LI**

(56) Entgegenhaltungen:
**FR - A - 2 267 323**
**GB - A - 2 075 042**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG, Klybeckstrasse 141, CH-4002 Basel (CH)**

(72) Erfinder: **Bedekovic, Davor, Dr., Stefanstrasse 12, CH-4106 Therwil (CH)**
Erfinder: **Fletcher, Ian John, Dr., Bürgenstal 27, CH-4312 Magden (CH)**

**EP 0 082 822 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft chromogene Dihydrofuropyridinone, Verfahren zu ihrer Herstellung und ihre Verwendung als Farbbildner in druckempfindlichen oder wärmeempfindlichen Aufzeichnungsmaterialien.

Azaphthalidverbindungen (=Dihydrofuropyridinone), welche in 3-Stellung einen basisch substituierten Phenylrest und einen N-substituierten Indol-3-ylrest aufweisen, sind beispielsweise in der FR-A 2 267 323, Beispiel 28 und in der GB-A-2 075 042 beschrieben.

Die erfindungsgemässen Dihydrofuropyridinone weisen einen durch einen Alkylrest mit 6 bis 9 C-Atomen N-substituierten Indolylrest auf und entsprechen der allgemeinen Formel (I)

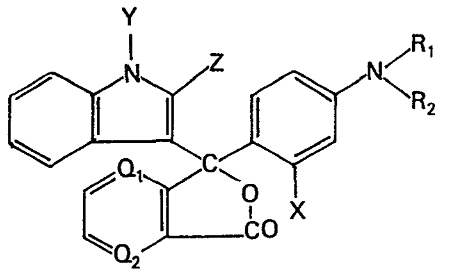
I

worin
$R_1$ und $R_2$ unabhängig voneinander Wasserstoff, $C_1$-$C_{12}$-Alkyl, β-Cyanoethyl, β-Chlorethyl, β-Hydroxyethyl, β-Methoxyethyl, β-Ethoxyethyl, $C_5$-$C_6$-Cycloalkyl oder unsubstituiertes oder durch Halogen, Methyl oder Methoxy substituiertes Benzyl oder Phenyl sind, oder $-NR_1R_2$ Pyrrolidinyl bedeutet und
X Wasserstoff, Halogen, $C_1$-$C_5$-Alkyl, $C_1$-$C_5$-Alkoxy oder Benzyloxy,
Y Alkyl mit 6 bis 9 Kohlenstoffatomen sowie
Z $C_1$-$C_5$-Alkyl ist und von $Q_1$ und $Q_2$ eines N und das andere CH bedeuten.

Die $C_1$-$C_5$-Alkyl und $C_1$-$C_5$-Alkoxy stellen bei der Definition der Reste der Dihydrofuropyridinone solche Gruppen oder Gruppenbestandteile dar, die vorzugsweise 1 bis 3 Kohlenstoffatome aufweisen, wie z.B. Methyl, Ethyl, n-Propyl, Isopropyl, aber auch n-Butyl, sek.-Butyl oder Amyl bzw. Methoxy, Ethoxy oder Isopropoxy.

Stellen die Substituenten $R_1$ und $R_2$ Alkylgruppen dar, so können sie geradkettige oder verzweigte Alkylreste sein. Beispiele für solche Alkylreste sind Methyl, Ethyl, n-Propyl, Isopropyl, n-Butyl, sek.-Butyl, Amyl, n-Hexyl, n-Octyl oder n-Dodecyl.

Beispiele für Cycloalkyl in der Bedeutung der R-Reste sind Cyclopentyl oder vorzugsweise Cyclohexyl.

Beispiele für substituiertes Benzyl oder Phenyl in der Bedeutung von $R_1$ und $R_2$ sind p-Methylbenzyl, o- oder p-Chlorbenzyl, o- oder p-Tolyl, Xylyl, o-, m- oder p-Chlorphenyl oder o- oder p-Methoxyphenyl.

Die Substituenten $R_1$ und $R_2$ sind vorzugsweise Benzyl oder in erster Linie $C_1$-$C_5$-Alkyl. $R_1$ und $R_2$ können zusammen mit dem sie verbindenden Stickstoffatom einen Pyrrolidinylrest bilden, der ebenfalls einen bevorzugten Substituenten darstellt. $R_1$ ist bevorzugt auch Cyclohexyl. X kann Wasserstoff, Halogen, $C_1$-$C_5$-Alkyl, wie z.B. Methyl; Benzyloxy, oder $C_1$-$C_5$-Alkoxy, wie z.B. Methoxy, Ethoxy, Isopropoxy oder tert.-Butoxy sein. Vorzugsweise ist X Wasserstoff, Benzyloxy oder $C_1$-$C_5$-Alkoxy und vor allem Ethoxy.

Als Alkylrest ist Y beispielsweise n-Hexyl, 2-Ethylhexyl, n-Heptyl, n-Octyl, Isooctyl, tert.-Octyl, n-Nonyl oder Isononyl.

Der N-Substituent Y ist vorzugsweise Alkyl mit 6 bis 8 Kohlenstoffatomen, z.B. n-Hexyl, n-Heptyl oder vor allem n-Octyl.

Z ist insbesondere Methyl.

Vorzugsweise stellen die erfindungsgemässen chromogenen Dihydrofuropyridinone Isomerengemische von 5,5-disubstituierten-5,7-dihydro-furo-7-pyridinonen und 7,7-disubstituierten-5,7-dihydro-furo-5-pyridinonen dar, wobei die Stickstoffatome des Pyridinringes in den angegebenen ortho-Stellungen angeordnet sind.

Praktisch wichtige chromogene Dihydrofuropyridinone entsprechen der Formel (II)

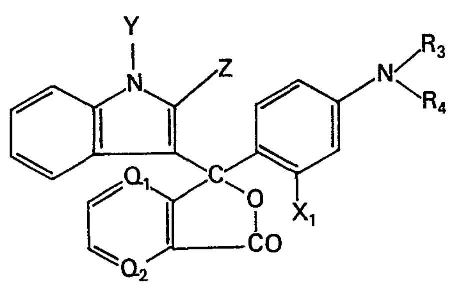
II

worin
$R_3$ und $R_4$ unabhängig voneinander $C_1$-$C_5$-Alkyl oder unsubstituiertes oder durch Halogen, Methyl oder Methoxy substituiertes Benzyl, oder $-NR_3R_4$ Pyrrolidinyl, von $Q_1$ und $Q_2$ eines N und das andere CH,
$X_1$ Wasserstoff, $C_1$-$C_5$-Alkoxy oder Benzyloxy,
Y Alkyl mit 6 bis 9 Kohlenstoffatomen, und
Z $C_1$-$C_5$-Alkyl bedeuten.

Halogen in Verbindung mit den vorstehenden Substituenten in Formeln (I) und (II) bedeutet beispielsweise Fluor, Brom oder vorzugsweise Chlor.

Unter den Dihydrofuropyridinonen der Formel (II), die vorzugsweise in Form von Isomerengemischen vorliegen, sind diejenigen, in denen
$X_1$ $C_1$-$C_5$-Alkoxy und
Y Alkyl mit 6 bis 8 Kohlenstoffatomen, vor allem n-Octyl bedeuten, bevorzugt. $R_3$ und $R_4$ sind vorzugsweise $C_1$-$C_5$-Alkyl.

Von ganz besonderem Interesse sind Isomerengemische von Dihydrofuropyridinonen der Formel (III)

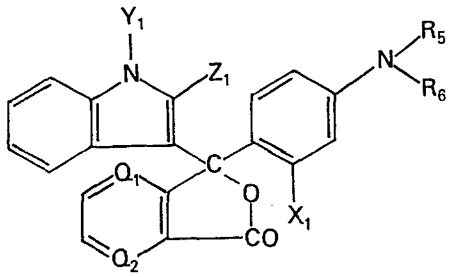

III

worin

$R_5$ und $R_6$ je $C_1$-$C_5$-Alkyl oder Benzyl, oder $-NR_5R_6$ Pyrrolidinyl, von $Q_1$ und $Q_2$ eines N und das andere CH,

$X_1$ Wasserstoff, Benzyloxy oder $C_1$-$C_5$-Alkoxy, insbesondere Ethoxy,

$Z_1$ Methyl und

$Y_1$ n-Hexyl oder vor allem n-Octyl bedeuten.

Unter diesen Verbindungen der Formel (III) sind diejenigen besonders bevorzugt, bei den $R_5$ und $R_6$ Methyl oder Ethyl oder $-NR_5R_6$ Pyrrolidinyl und $X_1$ Ethoxy, $Z_1$ Methyl und $Y_1$ n-Octyl bedeuten.

Die erfindungsgemässen Dihydrofuropyridinone der Formeln (I) bis (III) stellen neue Verbindungen dar und können nach an sich bekannten Methoden hergestellt werden. Ein Verfahren zur Herstellung der Dihydrofuropyridinone der Formel (I) besteht darin, dass man eine Verbindung der Formel (IV)

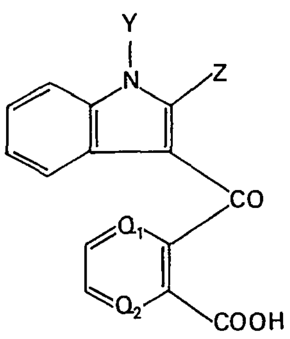

IV

mit einer Verbindung der Formel (V)

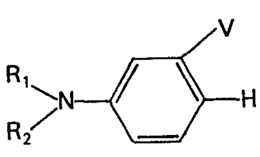

V

umsetzt, worin B, $Q_1$, $Q_2$, Y, Z, $R_1$ und $R_2$ die angegebene Bedeutung haben und V die für X angegebene Bedeutung hat oder Hydroxy bedeutet und das Reaktionsprodukt noch definitionsgemäss alkyliert oder aralkyliert, wenn V Hydroxy bedeutet.

Andererseits können die erfindungsgemässen Dihydrofuropyridinone auch dadurch hergestellt werden, dass man eine Verbindung der Formel (VI)

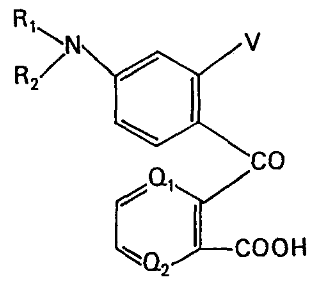

VI

mit einer Indolverbindung der Formel (VII)

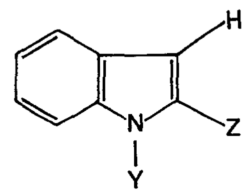

VII

umsetzt, worin $Q_1$, $Q_2$, $R_1$, $R_2$, Y und Z die angegebene Bedeutung haben und V die für X angegebene Bedeutung hat oder Hydroxy bedeutet, und das Reaktionsprodukt noch definitionsgemäss alkyliert oder aralkyliert, wenn V Hydroxy bedeutet.

Die Umsetzungen werden vorzugsweise so durchgeführt, dass man die Reaktionskomponenten in Anwesenheit eines sauren Entwässerungsmittels bei einer Temperatur von 20° bis 80°C zur Reaktion bringt. Beispiele für derartige Kondensationsmittel sind Essigsäureanhydrid, Schwefelsäure, Zinkchlorid und Phosphoroxychlorid.

Die Alkylierung oder Aralkylierung der Reaktionsprodukte, bei denen V Hydroxy bedeutet, wird in der Regel nach bekannten Verfahren durchgeführt. Beispielsweise wird die Reaktion in Anwesenheit eines säurebindenden Mittels, wie z.B. eines Alkalicarbonats oder einer tertiären Stickstoffbase, wie Triethylamin und gegebenenfalls in Anwesenheit eines inerten, organischen Lösungsmittels, wie z.B. Aceton, Isopropylalkohol, Chlorbenzol oder Nitrobenzol, durchgeführt. Geeignete Alkylierungsmittel sind Alkylhalogenide, wie z.B. Methyl- oder Ethyljodid oder -chlorid, oder Dialkylsulfate, wie Dimethyl- oder Diethylsulfat. Als Aralkylierungsmittel eignet sich insbesondere Benzylchlorid und die entsprechenden Substitutionsprodukte, wie z.B. p-Chlorbenzylchlorid oder 2,4-Dimethylbenzylchlorid, die vorzugsweise in einem nicht-polaren, organischen Lösungsmittel, wie z.B. Benzol, Toluol oder Xylol, verwendet werden.

Die Ausgangsstoffe der Formeln (IV) und (VI) werden in der Regel durch Umsetzung eines Anhydrids der Formel (VIII)

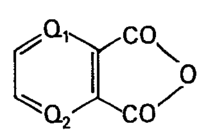

VIII

mit einere Verbindung der Formel (VII) bzw. mit einer Verbindung der Formel (V) erhalten, wobei die Reaktion gewünschtenfalls in einem organischen Lösungsmittel und gegebenenfalls in Anwesenheit einer Lewissäure, z.B. Aluminiumchlorid, durchgeführt wird. Geeignete organische Lösungsmittel sind z.B. Dimethylformamid, Acetonitril, niedere aliphatische Carbonsäuren, wie Essigsäure; Benzol, Toluol, Xylol oder Chlorbenzol. Die Reaktion wird vorzugsweise bei Temperaturen von 15°C bis zum Siedepunkt des eingesetzten Lösungsmittels durchgeführt. Die erhaltenen Verbindungen der Formel (IV) können, ohne isoliert zu werden, zur Umsetzung mit den Anilinverbindungen der Formel (V) weiterverwendet werden. Die Verbindungen der Formel (VI), worin V Alkoxy oder Benzyloxy darstellt, werden vorzugsweise durch Alkylierung bzw. Aralkylierung des Zwischenproduktes nach üblichen Methoden hergestellt, das durch Umsetzung eines Anhydrids der Formel (VIII) mit einer Anilinverbindung der Formel (V) erhalten wird, worin V Hydroxy ist. Die Alkylierungs- und Aralkylierungsmittel können die gleichen sein, wie sie für die Herstellung der Verbindungen der Formeln (I) bis (III) angegeben sind.

Die Dihydrofuropyridinone der Formeln (I) bis (III) sind normalerweise farblos oder höchstens schwach gefärbt. Wenn diese Farbbildner mit einem vorzugsweise sauren Entwickler, d.h. einem Elektronenakzeptor, in Kontakt gebracht werden, so ergeben sie intensive grün-blaue, blaue oder violett-blaue Farbtöne, die ausgezeichnet sublimations- und lichtecht sind. Sie sind deshalb auch sehr wertvoll im Gemisch mit einem oder mehreren anderen bekannten Farbbildnern, z.B. 3,3-(Bis-aminophenyl-)phthaliden, 3,3-(Bis-indolyl-)phthaliden, 3-Aminofluoranen, 2,6-Diaminofluoranen, Leukoauraminen, Spiropyranen, Spirodipyranen, Chromenoindolen, Phenoxyzinen, Phenothiazinen, Carbazolylmethanen oder weiteren Triarylmethan-leukofarbstoffen, um blaue, marine-blaue, graue oder schwarze Färbungen zu ergeben.

Die Dihydrofuropyridinone der Formeln (I) bis (III) zeigen sowohl auf Tonen, wie auch besonders auf phenolischen Unterlagen eine verbesserte Farbintensität und Lichtechtheit. Sie eignen sich vor allem als sich schnell entwickelnde Farbbildner für die Verwendung in einem wärmeempfindlichen oder insbesondere druckempfindlichen Aufzeichnungsmaterial, das sowohl Kopierals auch Registriermaterial sein kann.

Ein druckempfindliches Material besteht beispielsweise aus mindestens einem Paar von Blättern, die mindestens einen Farbbildner der Formeln (I) bis (III) gelöst in einem organischen Lösungsmittel und einen Elektronenakzeptor als Entwickler enthalten.

Typische Beispiele für solche Entwickler sind Aktivton-Substanzen, wie Attapulgus-Ton, Säureton, Bentonit, Montmorillonit, aktivierter Ton, wie z.B. säureaktiviertes Bentonit oder Montmorillonit, ferner Zeolith, Halloysit, Siliciumdioxyd, Aluminiumoxid, Aluminiumsulfat, Aluminiumphosphat, Zinkchlorid, aktiviertes Kaolin oder irgendein beliebiger Ton. Bevorzugte Entwickler sind sauer reagierende, organische Verbindungen, wie z.B. gegebenenfalls ringsubstituierte Phenole, Salicylsäure oder Salicylsäureester und deren Metallsalze, ferner ein sauer reagierendes, polymeres Material, wie z.B. ein phenolisches Polymerisat, ein Alkylphenolacetylenharz, ein Maleinsäure-Kolophonium-Harz oder ein teilweise oder vollständig hydrolysiertes Polymerisat von Maleinsäureanhydrid mit Styrol, Ethylen oder Vinylmethylether, oder Carboxypolymethylen. Es können auch Mischungen der genannten polymeren Verbindungen eingesetzt werden. Besonders bevorzugte Entwickler sind Zinksalicylate oder die Kondensationsprodukte von p-substituierten Phenolen mit Formaldehyd. Die letzteren können auch Zink enthalten.

Die Entwickler können zusätzlich auch mit anderen, an sich unreaktiven oder wenig reaktiven Pigmenten gemischt eingesetzt werden. Beispiele für solche Pigmente sind: Talk, Titandioxid, Zinkoxid, Kreide, Tone wie Kaolin, sowie organische Pigmente, z.B. Harnstoff-Formaldehyd- oder Melamin-Formaldehyd-Kondensationsprodukte.

Der Farbbildner liefert an den Punkten, an denen er mit dem Elektronenakzeptor in Kontakt kommt, eine gefärbte Markierung. Um zu verhindern, dass die Farbbildner, die in dem druckempfindlichen Aufzeichnungsmaterial enthalten sind, frühzeitig aktiv werden, werden sie in der Regel von dem Elektronenakzeptor getrennt. Dies kann zweckmässig erzielt werden, indem man die Farbbildner in schaum-, schwamm- oder bienenwabenartigen Strukturen einarbeitet. Vorzugsweise sind die Farbbildner in Mikrokapseln eingeschlossen, die sich in der Regel durch Druck zerbrechen lassen.

Wenn die Kapseln durch Druck, beispielsweise mittels eines Bleistiftes zerbrochen werden und wenn die Farbbildnerlösung auf diese Weise auf ein benachbartes Blatt übertragen wird, das mit einem Elektronenakzeptor beschichtet ist, wird eine farbige Stelle erzeugt. Diese Farbe resultiert aus dem dabei gebildeten Farbstoff, der im sichtbaren Bereich des elektromagnetischen Spektrums absorbiert.

Die Farbbildner werden vorzugsweise in Form von Lösungen in organischen Lösungsmitteln eingekapselt. Beispiele für geeignete Lösungsmittel sind vorzugsweise nichtflüchtige Lösungsmittel, z.B. polyhalogeniertes Paraffin oder Diphenyl, wie Chlorparaffin, Monochlordiphenyl oder Trichlordiphenyl, ferner Tricresylphosphat, Di-n-butylphthalat, Dioctylphthalat, Trichlorbenzol, Trichlorethylphosphat, aromatische Ether, wie Benzylphenylether, Kohlenwasserstofföle, wie Paraffin oder Kerosin, z.B. mit Isopropyl, Isobutyl, sek-Butyl oder tert.-Butyl alkylierte Derivate von Diphenyl, Naphthalin oder Triphenyl, Dibenzyltoluol, Terphenyl, partiell hydriertes Terphenyl, mono- und/oder dibenzylierte Xylole, mono- bis tetramethylierte Diphenylalkane, z.B. Bis-Tolylethan oder Bis-Xylylethan oder weitere chlorierte oder hydrierte kondensierte, aromatische Kohlenwasserstoffe. Oft werden Mischungen verschiedener Lösungsmittel, insbesondere Mischungen aus Paraffinölen oder Kerosin und

Diisopropylnaphthalin oder partiell hydriertem Terphenyl, eingesetzt, um eine optimale Löslichkeit für die Farbbildung, eine rasche und intensive Färbung und eine für die Mikroverkapselung günstige Viskosität zu erreichen. Diesbezüglich zeichnen sich die erfindungsgemässen Dihydrofuropyridinone dadurch aus, dass sie dank dem langkettigen N-Alkylrest von Y in den Lösungen der genannten Lösungsmittel, insbesondere Diisopropylnaphthalin oder partiell hydrierten Terphenyl bei einem pH-Bereich von 4 bis 10 farblos bleiben und somit die wässerige Phase z.B. bei der Einkapselung nicht anfärben.

Die Kapselwände können durch Koazervationskräfte gleichmässig um die Tröpfchen der Farbbildnerlösung herum gebildet werden, wobei das Einkapselungsmaterial z.B. aus Gelatine und Gummi arabicum bestehen kann, wie dies z.B. in der US-A-2 800 457 beschrieben ist. Die Kapseln können vorzugsweise auch aus einem Aminoplast oder modifizierten Aminoplasten durch Polykondensation gebildet werden, wie es in den GB-A-989,264, 1 156 725, 1 301 052 und 1 355 124 beschrieben ist. Ebenfalls geeignet sind Mikrokapseln, welche durch Grenzflächenpolymerisation gebildet werden, wie z.B. Kapseln aus Polyester, Polycarbonat, Polysulfonamid, Polysulfonat, besonders aber aus Polyamid oder Polyurethan.

Die Farbbildner der Formeln (I) bis (III) enthaltenden Mikrokapseln können zur Herstellung von druckempfindlichen Kopiermaterialien der verschiedensten bekannten Arten verwendet werden. Die verschiedenen Systeme unterscheiden sich im wesentlichen voneinander durch die Anordnung der Kapseln, der Farbreaktanten und durch das Trägermaterial. Bevorzugt wird eine Anordnung, bei der der eingekapselte Farbbildner in Form einer Schicht auf der Rückseite eines Übertragungsblattes und der Elektronenakzeptor in Form einer Schicht auf der Vorderseite eines Empfangsblattes vorhanden sind. Eine andere Anordnung der Bestandteile besteht darin, dass die den Farbbildner enthaltenden Mikrokapseln und der Entwickler in oder auf dem gleichen Blatt in Form einer oder mehrerer Einzelschichten oder in der Papierpulpe vorliegen.

Die Kapseln werden vorzugsweise mittels eines geeigneten Binders auf dem Träger befestigt. Da Papier das bevorzugte Trägermaterial ist, handelt es sich bei diesem Binder hauptsächlich um Papierbeschichtungsmittel, wie Gummi arabicum, Polyvinylalkohol, Hydroxymethylcellulose, Casein, Methylcellulose, Dextrin, Stärke oder Stärkederivate oder Polymerlatices. Letztere sind beispielsweise Butadien-Styrolcopolymerisate oder Acrylhomo- oder -Copolymere. Als Papier werden nicht nur normale Papiere aus Cellulosefasern, sondern auch Papiere, in denen die Cellulosefasern (teilweise oder vollständig) durch Fasern aus synthetischen Polymerisaten ersetzt sind, verwendet.

Die Verbindungen der Formeln (I) bis (III) können auch als Farbbildner in einem thermoreaktiven Aufzeichnungsmaterial verwendet werden. Dieses enthält in der Regel mindestens einen Schichtträger, einen Farbbildner, einen Elektronenakzeptor und gegebenenfalls auch ein Bindemittel.

Thermoreaktive Aufzeichnungssysteme umfassen z.B. wärmeempfindliche Aufzeichnungs- und Kopiermaterialien und -papiere. Diese Systeme werden beispielsweise zum Aufzeichnen von Informationen, z.B. in elektronischen Rechnern, Ferndruckern, Fernschreibern oder in Aufzeichnungsgeräten und Messinstrumenten, wie z.B. Elektrocardiographen verwendet. Die Bilderzeugung (Markierung) kann auch manuell mit einer erhitzten Feder erfolgen. Eine weitere Einrichtung der Erzeugung von Markierungen mittels Wärme sind Laserstrahlen.

Das Thermoreaktive Aufzeichnungsmaterial kann so aufgebaut sein, dass der Farbbildner in einer Bindemittelschicht gelöst oder dispergiert ist und in einer zweiten Schicht der Entwickler in dem Bindemittel gelöst und dispergiert ist. Eine andere Möglichkeit besteht darin, dass sowohl der Farbbildner als auch der Entwickler in einer Schicht dispergiert sind. Das Bindemittel wird in spezifischen Bezirken mittels Wärme erweicht und an diesen Punkten, an denen Wärme angewendet wird, kommt der Farbbildner mit dem Elektronenakzeptor in Kontakt und es entwickelt sich sofort die erwünschte Farbe.

Als Entwickler eignen sich die gleichen Elektronenakzeptoren, wie sie in druckempfindlichen Papieren verwendet werden. Beispiele für Entwickler sind die bereits erwähnten Tonminerale und besonders Phenolharze, oder auch phenolische Verbindungen, wie sie beispielsweise in der DE-C-1 251 348 beschrieben sind, z.B. 4-tert.-Butylphenyl, 4-Phenylphenol, 4-Hydroxydiphenyläther, α-Naphthol, β-Naphthol, 4-Hydroxybenzoesäuremethylester. 4-Hydroxyacetophenon, 2,2′-Dihydroxydiphenyl, 4,4′-Isopropylidendiphenol, 4,4′-Isopropyliden-bis-(2-methylphenol), 4,4′-Bis-(hydroxyphenyl)valeriansäure, 2,2′-Methylen-bis-(4-phenylphenol), Hydrochinon, Pyrogallol, Phloroglucin, p-, m-, o-Hydroxybenzoesäure, Gallussäure, 1-Hydroxy-2-naphthoesäure sowie Borsäure und organische vorzugsweise aliphatische Dicarbonsäuren, wie z.B. Weinsäure, Oxalsäure, Maleinsäure, Zitronensäure, Citraconsäure und Bernsteinsäure.

Vorzugsweise werden zur Herstellung des thermoreaktiven Aufzeichnungsmaterials schmelzbare, filmbildende Bindemittel verwendet. Diese Bindemittel sind normalerweise wasserlöslich, während die Dihydrofuropyridinone und der Entwickler in Wasser schwer löslich oder unlöslich sind. Das Bindemittel sollte in der Lage sein, den Farbbildner und den Entwickler bei Raumtemperatur zu dispergieren und zu fixieren.

Bei Einwirkung von Wärme erweicht oder schmilzt das Bindemittel, so dass der Farbbildner mit dem Entwickler in Kontakt kommt und sich eine Farbe bilden kann. Wasserlösliche oder mindestens in Wasser quellbare Bindemittel sind z.B. hydrophile Polymerisate, wie Polyvinylalkohol, Polyacrylsäure, Hydroxyethylcellulose, Methylcellulose, Carboxymethylcellulose, Polyacrylamid, Polyvinylpyrrolidon, Gelatine oder Stärke.

Wenn der Farbbildner und der Entwickler in zwei getrennten Schichten vorliegen, können in Wasser unlösliche Bindemittel, d.h. in nichtpolaren oder nur schwach polaren Lösungsmitteln lösliche Bindemittel, wie z.B. Naturkautschuk, synthetischer Kautschuk, chlorierter Kautschuk, Alkydharze, Polystyrol, Styrol/Butadien-Mischpolymerisate, Polymethylacrylate, Ethylcellulose, Nitrocellulose und Polyvinylcarbazol, verwendet werden. Die bevorzugte Anordnung ist jedoch diejenige, bei der der Farbbildner und der Entwickler in einer Schicht in einem wasserlöslichen Bindemittel enthalten sind.

Die thermoreaktiven Schichten können weitere Zusätze enthalten. Zur Verbesserung des Weissgrades, zur Erleichterung des Bedruckens der Papiere und zur Verhinderung des Festklebens der erhitzten Feder können diese Schichten, z.B. Talk, Titandioxyd, Zinkoxyd, Aluminiumhydroxid, Calciumcarbonat (z.B. Kreide), Tone oder auch organische Pigmente, wie z.B. Harnstoff-Formaldehydpolymerisate, enthalten. Um zu bewirken, dass nur innerhalb eines begrenzten Temperaturbereiches die Farbe gebildet wird, können Substanzen, wie Harnstoff, Thioharnstoff, Diphenylthioharnstoff, Acetamid, Acetanilid, Stearinsäureamid, Phthalsäureanhydrid, Metallstearate, Phthalsäurenitril oder andere entsprechende, schmelzbare Produkte, welche das gleichzeitige Schmelzen des Farbbildners und des Entwicklers induzieren, zugesetzt werden. Bevorzugt enthalten thermographische Aufzeichnungsmaterialien Wachse, z.B. Carnaubawachs, Montanawachs, Paraffinwachs oder Polyethylenwachs.

In den folgenden Beispielen beziehen sich die angegebenen Prozentsätze, wenn nichts anderes angegeben ist, auf das Gewicht.

Beispiel 1

60 g Chinolinsäureanhydrid und 116 g 1-n-Octyl-2-methylindol werden während 3 1/2 Stunden bei 65 bis 70°C in 100 ml Toluol verrührt. Hierauf wird die Reaktionsmischung zur Trockene eingedampft und der Rückstand in 2 l Ethanol bei 70°C gelöst. Man kühlt die Lösung auf 0°C ab, wobei das Produkt ausfällt. Dieses wird dann abfiltriert, mit Ethanol und Petrolether gewaschen und im Vakuum bei 60°C getrocknet. Man erhält 90 g (57% der Theorie) eines Isomerengemisches bestehend aus den Verbindungen der Formeln (A) und (B)

A 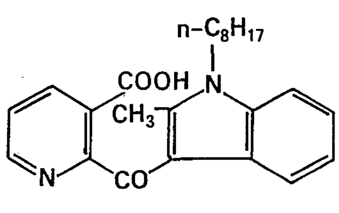 und

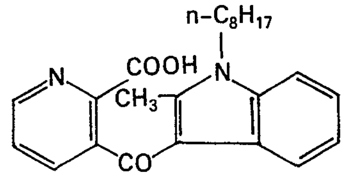 B

das bei 105 bis 112°C schmilzt.

39,3 g dieses Isomerengemisches werden mit 19,3 g 3-Diethylaminophenetol in 130 ml Essigsäureanhydrid während 3 1/2 Stunden bei 60 bis 65°C verrührt. Danach wird die Reaktionsmischung auf 1 l Wasser gegossen und unter Rühren durch Zugabe einer 30%igen Natriumhydroxidlösung auf pH 8 gestellt. Das ausgeschiedene Öl wird abgetrennt und in Toluol gelöst. Die Tolullösung wird über Natriumsulfat getrocknet und eingedampft. Der ölige Rückstand wird durch eine Aluminiumoxidsäule mit einer Mischung aus Chloroform und Methanol (1:1) chromatographiert. Man erhält 30 g (53% der Theorie) eines Isomerengemisches bestehend aus Verbindungen der Formeln (C) und (D)

C 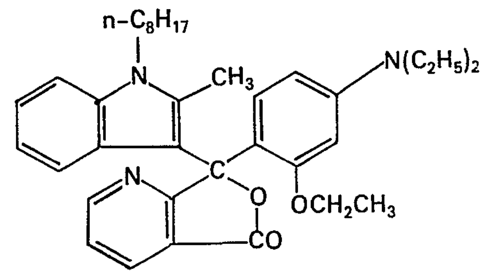

und

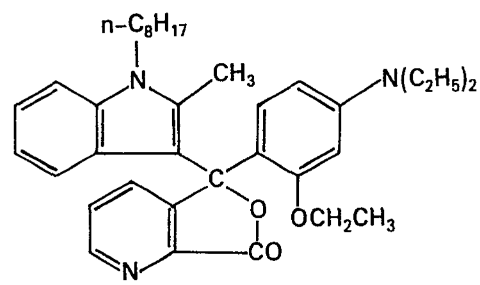 D

mit einem Schmelzpunkt von 119 bis 121°C. Auf Phenolharz entwickelt dieser Farbbildner eine blaue Farbe.

Auf die gleiche Art und Weise wie in Beispiel 1 beschrieben erhält man unter Verwendung der entsprechenden Ausgangsstoffe Isomerengemische der in der folgenden Tabelle aufgeführten Dihydrofuropyridinone der Formeln (E) und (F)

E 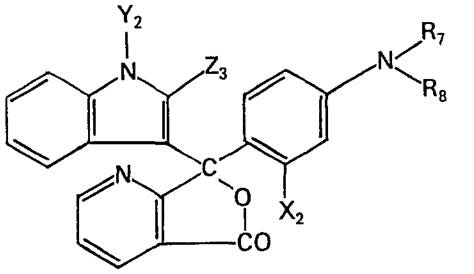 und 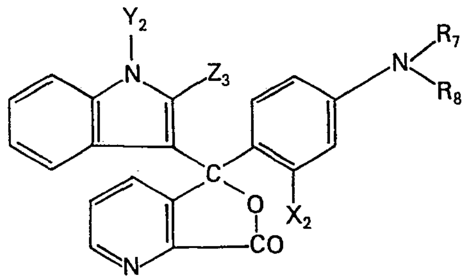 F

Tabelle

| Beispiel | 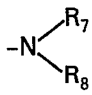 | $X_2$ | $Y_2$ | $Z_3$ | Smp./°C | Farbe auf Phenolharz |
|---|---|---|---|---|---|---|
| 2 | $-N(C_2H_5)_2$ | $-OC_2H_5$ | $-n-C_6H_{13}$ | $-CH_3$ | 134–136 | blau |
| 3 | $-N(CH_3)_2$ | H | $-n-C_8H_{17}$ | $-CH_3$ | 126–128 | blau |
| 4 | $-N(C_2H_5)_2$ | $-OC_2H_5$ | $-n-C_7H_{15}$ | $-CH_3$ | 109–113 | blau |
| 5 | $-N(C_2H_5)_2$ | $-OC_2H_5$ | $-n-C_9H_{19}$ | $-CH_3$ | 105–107 | blau |
| 6 | $-N(C_2H_5)_2$ | $-OCH_2$ 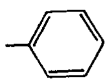 | $-n-C_8H_{17}$ | $-CH_3$ | 166–167 | blau |
| 7 | 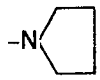 | $-OC_2H_5$ | $-n-C_6H_{13}$ | $-CH_3$ | 146–148 | blau |
| 8 | 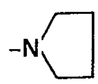 | $-OC_2H_5$ | $-n-C_7H_{15}$ | $-CH_3$ | 151–153 | blau |
| 9 | 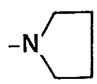 | $-OC_2H_5$ | $-n-C_8H_{17}$ | $-CH_3$ | 147–148 | blau |
| 10 | 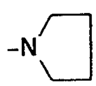 | $-OC_2H_5$ | $-n-C_9H_{19}$ | $-CH_3$ | 115–118 | blau |
| 11 | 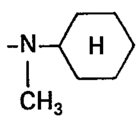 | $-OC_2H_5$ | $-n-C_8H_{17}$ | $-CH_3$ | | blau |

Herstellung eines druckempfindlichen Kopierpapiers

Beispiel 12

Eine Lösung von 3 g des Isomerengemisches von den Dihydrofuropyridinonen der Formeln (C) und (D) (Beispiel 1) in 80 g Diisopropylnaphthalin und 17 g Kerosin wird auf an sich bekannte Weise mit Gelatine und Gummi arabicum durch Koazervation mikroverkapselt, mit Stärkelösung vermischt und auf ein Blatt Papier gestrichen. Ein zweites Blatt Papier wird auf der Frontseite mit Phenolharz als Farbentwickler beschichtet. Das erste Blatt und das mit Farbentwickler beschichtete Papier werden mit den Beschichtungen benachbart aufeinandergelegt. Durch Schreiben mit der Hand oder mit der Schreibmaschine auf dem ersten Blatt wird Druck ausgeübt, und es entwickelt sich sofort auf dem mit dem Entwickler beschichteten Blatt eine intensive blaue Kopie, die ausgezeichnet lichtecht ist.

Entsprechende intensive und lichtechte blaue Kopien werden auch bei Verwendung jedes der anderen in den Herstellungs-Beispielen 2 bis 11 angegebenen Farbbildner erzielt.

Beispiel 13

1 g des Isomerengemisches der Dihydrofuropyridinone der Formeln (C) und (D) wird in 17 g Toluol gelöst. Zu dieser Lösung gibt man unter Rühren 12 g Polyvinylacetat, 8 g Calciumcarbonat und 2 g Titandioxid. Die erhaltene Suspension wird im Gewichtsverhältnis 1:1 mit Toluol verdünnt und mit einem 10 µm Rakel auf ein Blatt Papier gestrichen. Auf dieses Blatt Papier wird ein zweites Blatt Papier gelegt, dessen Unterseite bei einem Auftragsgewicht von 3 g/m² mit einer Mischung bestehend aus 1 Teil eines Amidwachses,

1 Teil eines Stearinwachses und 1 Teil Zinkchlorid beschichtet ist. Durch Schreiben mit der Hand oder mit der Schreibmaschine auf dem oberen Blatt wird Druck ausgeübt, und es entwickelt sich sofort auf dem mit dem Farbbildner beschichteten Blatt eine intensive und lichtechte blaue Farbe.

Herstellung eines wärmeempfindlichen Aufzeichnungsmaterials

Beispiel 14

In einer Kugelmühle werden 32 g 4,4′-Isopropyliden-diphenol (Bisphenol A), 3,8 g Distearylamid des Äthylendiamins, 39 g Kaolin, 20 g eines zu 88% hydrolysierten Polyvinylalkohols und 500 ml Wasser gemahlen bis die Teilchengrösse ca. 5 μm beträgt. In einer zweiten Kugelmühle werden 6 g des Isomerengemisches aus den Dihydrofuropyridinonen der Formeln (C) und (D) (Beispiel 1), 3 g eines zu 88% hydrolysierten Polyvinylalkohols und 60 ml Wasser zu einer Teilchengrösse von ca. 3 μm gemahlen.

Die beiden Dispersionen werden zusammengegeben und mit einem Trockenauftragsgewicht von 5,5 g/m² auf ein Papier gestrichen. Durch Berührung des Papiers mit einem erhitzten Kugelschreiber wird eine intensive blaue Farbe erhalten, die eine ausgezeichnete Licht- und Sublimierechtheit hat.

Intensive und lichtechte blaue Farben können auch bei Verwendung jeder der anderen Farbbildner gemäss Beispielen 2 bis 11 erhalten werden.

Beispiel 15

In einer Kugelmühle werden 2,7 g des Isomerengemisches der Dihydrofuropyrimidone der Formeln (C) und (D), 24 g N-Phenyl-N′-(1-hydroxy-2,2,2-trichloräthyl)-harnstoff, 16 g Stearinsäureamid, 59 g eines zu 88% hydrolysierten Polyvinylalkohols und 58 ml Wasser gemahlen bis die Teilchengrösse 2–5 μm beträgt. Diese Suspension wird bei einem Trockenauftragsgewicht von 5,5 g/m² auf ein Blatt Papier gestrichen. Durch Berührung des Papiers mit einem erhitzten Kugelschreiber wird eine intensive und lichtechte blaue Farbe erhalten.

**Patentansprüche**

1. Chromogene Dihydrofuropyridinone, dadurch gekennzeichnet, dass sie der allgemeinen Formel (I)

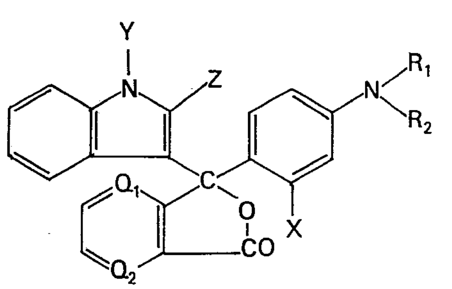

I

entsprechen, worin

$R_1$ und $R_2$ unabhängig voneinander Wasserstoff, $C_1$-$C_{12}$-Alkyl, β-Cyanoethyl, β-Chlorethyl, β-Hydroxyethyl, β-Methoxyethyl, β-Ethoxyethyl, $C_5$-$C_6$-Cycloalkyl oder unsubstituiertes oder durch Halogen, Methyl oder Methoxy substituiertes Benzyl oder Phenyl sind, oder $-NR_1R_2$ Pyrrolidinyl bedeutet,

X Wasserstoff, Halogen, $C_1$-$C_5$-Alkyl, $C_1$-$C_5$-Alkoxy oder Benzyloxy,

Y Alkyl mit 6 bis 9 Kohlenstoffatomen sowie

Z $C_1$-$C_5$-Alkyl ist und von $Q_1$ und $Q_2$ eines N und das andere CH bedeuten.

2. Dihydrofuropyridinone gemäss Anspruch 1, dadurch gekennzeichnet, dass in Formel (I) $R_1$ $C_1$-$C_5$-Alkyl, Benzyl oder Cyclohexyl und $R_2$ $C_1$-$C_5$-Alkyl oder Benzyl bedeuten oder $-NR_1R_2$ Pyrrolidinyl darstellt.

3. Dihydrofuropyridinone gemäss Anspruch 1 oder 2, dadurch gekennzeichnet, dass in Formel (I) X Wasserstoff, $C_1$-$C_5$-Alkoxy oder Benzyloxy bedeutet.

4. Dihydrofuropyridinone gemäss Anspruch 1, dadurch gekennzeichnet, dass sie der Formel (II)

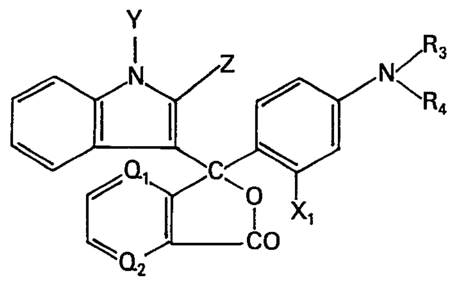

II

entsprechen, worin

$R_3$ und $R_4$ unabhängig voneinander $C_1$-$C_5$-Alkyl oder unsubstituiertes oder durch Halogen, Methyl oder Methoxy substituiertes Benzyl, oder $-NR_3R_4$ Pyrrolidinyl, von $Q_1$ und $Q_2$ eines N und das andere CH

$X_1$ Wasserstoff, $C_1$-$C_5$-Alkoxy oder Benzyloxy;

Y Alkyl mit 6 bis 9 Kohlenstoffatomen und

Z $C_1$-$C_5$-Alkyl bedeuten.

5. Dihydrofuropyridinone gemäss Anspruch 4, dadurch gekennzeichnet, dass in Formel (II)

$X_1$ $C_1$-$C_5$-Alkoxy und

Y Alkyl mit 6 bis 8 Kohlenstoffatomen vorzugsweise n-Octyl bedeuten.

6. Dihydrofuropyridinone gemäss Anspruch 4, dadurch gekennzeichnet, dass sie der Formel (III)

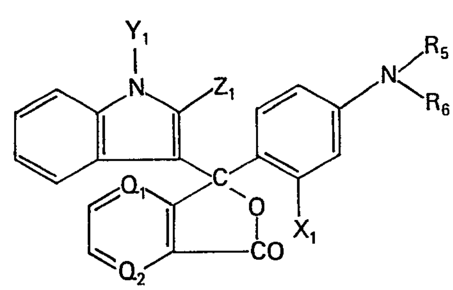

III

entsprechen, worin
$R_5$ und $R_6$ je $C_1$-$C_5$-Alkyl oder Benzyl, oder $-NR_5R_6$ Pyrrolidinyl, von $Q_1$ und $Q_2$ eines N und das andere CH,
$X_1$ Wasserstoff, Benzyloxy oder $C_1$-$C_5$-Alkoxy,
$Z_1$ Methyl und
$Y_1$ n-Hexyl oder n-Octyl bedeuten.

7. Dihydrofuropyridinone gemäss Anspruch 6, dadurch gekennzeichnet, dass in Formel (3) $R_5$ und $R_6$ Methyl oder Ethyl, $X_1$ Ethoxy, $Z_1$ Methyl und $Y_1$ n-Octyl bedeuten.

8. Dihydrofuropyridinone gemäss Anspruch 6, dadurch gekennzeichnet, dass in Formel (3) $-NR_5R_6$ Pyrrolidinyl, $X_1$ Ethoxy, $Z_1$ Methyl und $Y_1$ n-Octyl bedeuten.

9. Verfahren zur Herstellung von Dihydrofuropyridinonen gemäss Anspruch 1, dadurch gekennzeichnet, dass man eine Verbindung der Formel (IV)

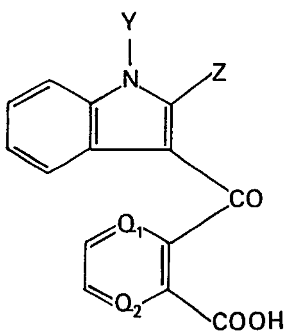

IV

mit einer Verbindung der Formel (V)

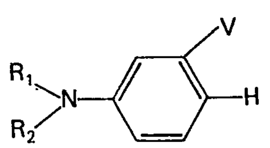

V

umsetzt, worin $Q_1$, $Q_2$, Y, Z, $R_1$ und $R_2$ die im Anspruch 1 angegebene Bedeutung haben und V die im Anspruch 1 für X angegebene Bedeutung hat oder Hydroxy bedeutet, und das Reaktionsprodukt mit einer geeigneten Verbindung noch alkyliert oder aralkyliert, wenn V Hydroxy bedeutet.

10. Verfahren zur Herstellung von Dihydrofuropyridinonen gemäss Anspruch 1, dadurch gekennzeichnet, dass man eine Verbindung der Formel (VI)

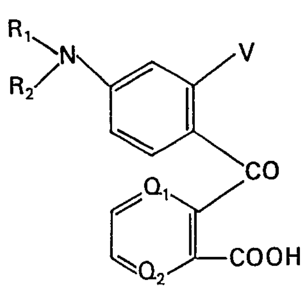

VI

mit einer Indolverbindung der Formel (VII)

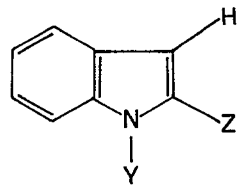

VII

umsetzt, worin $Q_1$, $Q_2$, $R_1$, $R_2$, Y und Z die im Anspruch 1 angegebene Bedeutung haben und V die im Anspruch 1 für X angegebene Bedeutung hat oder Hydroxy bedeutet, und das Reaktionsprodukt noch alkyliert oder aralkyliert, wenn V Hydroxy bedeutet.

11. Verwendung einer Dihydrofuropyridinonverbindung der in einem der Ansprüche 1 bis 8 angegebenen Formel als Farbbildner in einem druckempfindlichen oder wärmeempfindlichen Aufzeichnungsmaterial.

12. Druck- oder wärmeempfindliches Aufzeichnungsmaterial, dadurch gekennzeichnet, dass es in seinem Farbreaktantensystem als Farbbildner mindestens eine Dihydrofuropyridinonverbindung der in einem der Ansprüche 1 bis 8 angegebenen Formel enthält.

## Claims

1. A chromogenic dihydrofuropyridinone of the general formula (I)

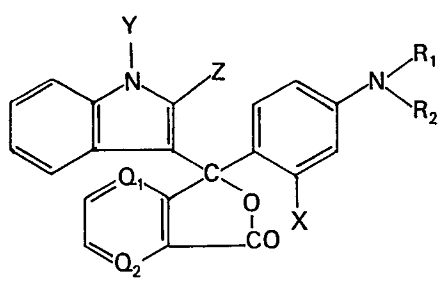

I

wherein
$R_1$ and $R_2$ are each independently of the other hydrogen, $C_1$-$C_{12}$alkyl, β-cyanoethyl, β-chloroethyl, β-hydroxyethyl, β-methoxyethyl, β-ethoxyethyl, $C_5$-$C_6$cycloalkyl, benzyl or phenyl, or benzyl or phenyl which are substituted by halogen, methyl or methoxy; or $-NR_1R_2$ is pyrrolidinyl;
X is hydrogen, halogen, $C_1$-$C_5$alkyl, $C_1$-$C_5$alkoxy or benzyloxy;
Y is $C_6$-$C_9$alkyl;
Z is $C_1$-$C_5$alkyl and
one of $Q_1$ and $Q_2$ is nitrogen and the other is CH.

2. A dihydrofuropyridinone according to claim 1, wherein in formula (I) $R_1$ is $C_1$-$C_5$alkyl benzyl or cyclohexyl and $R_2$ is $C_1$-$C_5$alkyl or benzyl; or $-NR_1R_2$ is pyrrolidinyl.

3. A dihydrofuropyridinone according to either of claims 1 or 2, wherein in formula (I) X is hydrogen, $C_1$-$C_5$alkoxy or benzyloxy.

4. A dihydrofuropyridinone according to claim 1 of the formula (II)

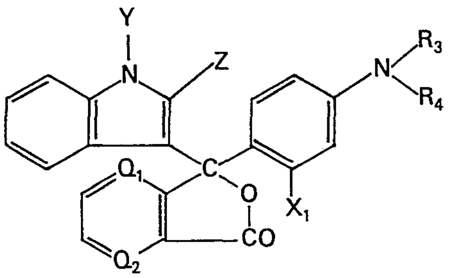

II

wherein

$R_3$ and $R_4$ are each independently of the other $C_1$-$C_5$alkyl, benzyl or benzyl which is substituted by halogen, methyl or methoxy; or $-NR_3R_4$ is pyrrolidinyl;

one of $Q_1$ and $Q_2$ is nitrogen and the other is CH;

$X_1$ is hydrogen, $C_1$-$C_5$-alkoxy or benzyloxy;

Y is $C_6$-$C_9$-alkyl and

Z is $C_1$-$C_5$-alkyl.

5. A dihydrofuropyridinone according to claim 4, wherein in formula (II)

$X_1$ is $C_1$-$C_5$-alkoxy and

Y is $C_6$-$C_8$-alkyl, preferably n-octyl.

6. A dihydrofuropyridinone according to claim 4 of the formula (III)

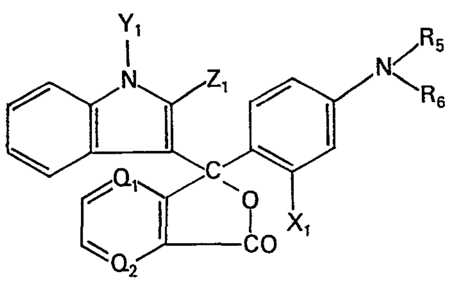

III

wherein

$R_5$ and $R_6$ are each independently of the other $C_1$-$C_5$-alkyl or benzyl; or $-NR_5R_6$ is pyrrolidinyl;

one of $Q_1$ and $Q_2$ is nitrogen and the other is CH;

$X_1$ is hydrogen, benzyloxy or $C_1$-$C_5$-alkoxy;

$Z_1$ is methyl and

$Y_1$ is n-hexyl or n-octyl.

7. A dihydrofuropyridinone according to claim 6, wherein in formula (III) $-NR_5R_6$ is pyrrolidinyl, $X_1$ is ethoxy, $Z_1$ is methyl and Y is n-octyl.

8. A dihydrofuropyridinone according to claim 6, wherein in formula (III) $-NR_5R_6$ is pyrrolidinyl, $X_1$ is ethoxy, $Z_1$ is methyl and $Y_1$ is n-octyl.

9. A process for the preparation of a dihydrofuropyridinone according to claim 1, which process comprises reacting a compound of the formula (IV)

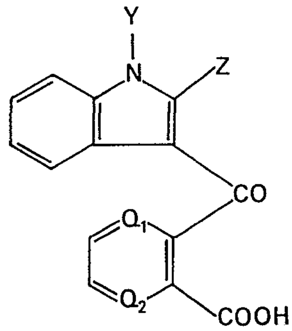

IV

with a compound of the formula (V)

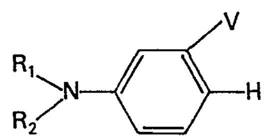

V

in which formulae $Q_1$, $Q_2$, Y, Z, $R_1$ and $R_2$ are as defined in claim 1 and V has the meaning of X as indicated in claim 1 or is hydroxy, and subsequently alkylating or aralkylating the reaction product with a suitable compound if V is hydroxy.

10. A process for the preparation of a dihydrofuropyridinone according to claim 1, which process comprises reacting a compound of the formula (VI)

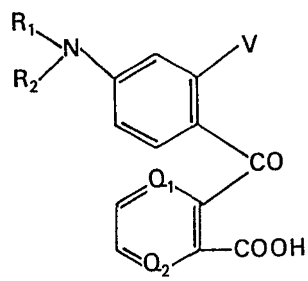

VI

with an indole compound of the formula (VII)

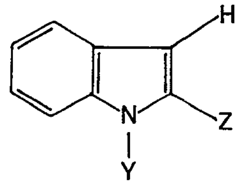

VII

in which formulae $Q_1$, $Q_2$, $R_1$, $R_2$, Y and Z are as defined in claim 1 and V has the meaning of X as indicated in claim 1 or is hydroxy, and subsequently alkylating or aralkylating the reaction product if V is hydroxy.

11. Use of a dihydrofuropyridinone compound of the formula as indicated in any one of claims 1

to 8 as colour former in a pressure-sensitive or heat-sensitive recording material.

12. A pressure-sensitive or heat-sensitive recording material which contais in its colour reactant system as colour former at least one dihydrofuropyridinone compound of the formula as indicated in any one of claims 1 to 8.

**Revendications**

1. Dihydrofuropyridinones chromogènes, caractérisées en ce qu'elles correspondent à la formule générale (I)

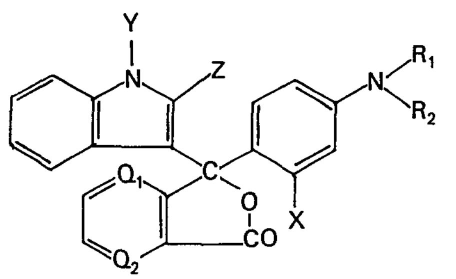

I

dans laquelle

$R_1$ et $R_2$, indépendamment l'un de l'autre, sont chacun l'hydrogène, un radical alkyle en $C_1$-$C_{12}$, β-cyanoéthyle, β-chloréthyle, β-hydroxyéthyle, β-méthoxyéthyle, β-éthoxyéthyle, cycloalkyle en $C_5$-$C_6$, ou benzyle ou phényle nonsubstitué ou substitué par un radical halogène, méthyle ou méthoxy, ou bien -$NR_1R_2$ est le radical pyrrolidinyle,

X est l'hydrogène, un halogène, un radical alkyle en $C_1$-$C_5$, alcoxy en $C_1$-$C_5$ ou benzyloxy,

Y est un radical alkyle ayant de 6 à 9 atomes de carbone, et

Z est un radical alkyle en $C_1$-$C_5$ et, entre les symboles $Q_1$ et $Q_2$, l'un désigne N et l'autre CH.

2. Dihydrofuropyridinones selon la revendication 1, caractérisées en ce que, dans la formule (I), $R_1$ est un radical alkyle en $C_1$-$C_5$, benzyle ou cyclohexyle, et $R_2$ est un radical alkyle en $C_1$-$C_5$ ou benzyle, ou bien –$NR_1R_2$ représente le radical pyrrolidinyle.

3. Dihydrofuropyridinones selon la revendication 1 ou 2, caractérisées en ce que, dans la formule (I), X est l'hydrogène, un radical alcoxy en $C_1$–$C_5$ ou benzyloxy.

4. Dihydrofuropyridinones selon la revendication 1, caractérisées en ce qu'elles correspondent à la formule (II)

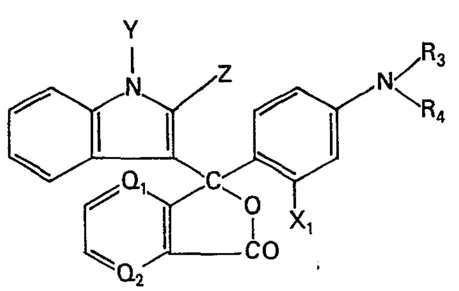

II

dans laquelle

$R_3$ et $R_4$, indépendamment l'un de l'autre, sont chacun un radical alkyle en $C_1$-$C_5$, ou benzyle non-substitué ou substitué par un halogène ou par un radical méthyle ou méthoxy, ou -$NR_3R_4$ représente un radical pyrrolidinyle,

des symboles $Q_1$ et $Q_2$, l'un représente N et l'autre CH,

$X_1$ est l'hydrogène, un radical alcoxy en $C_1$-$C_5$ ou benzyloxy,

Y est un radical alkyle ayant de 6 à 9 atomes de carbone, et

Z est un radical alkyle en $C_1$-$C_5$.

5. Dihydrofuropyridinones selon la revendication 4, caractérisées en ce que, dans la formule (II),

$X_1$ est un radical alcoxy en $C_1$-$C_5$, et Y est un radical alkyle ayant de 6 à 8 atomes de carbone, de préférence le radical n-octyle.

6. Dihydrofuropyridinones selon la revendication 4, caractérisées en ce qu'elles correspondent à la formule (III)

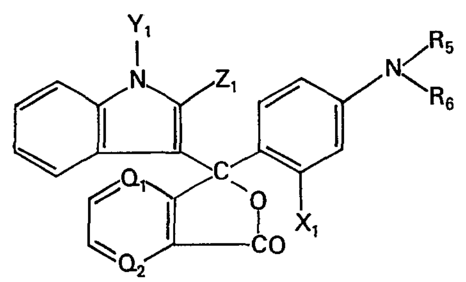

III

dans laquelle

$R_5$ et $R_6$ sont chacun un radical alkyle en $C_1$-$C_5$ ou benzyle, ou bien -$NR_5R_6$ représente le radical pyrrolidinyle, de $Q_1$ et $Q_2$, l'un est N et l'autre CH,

$X_1$ est l'hydrogène, le radical benzyloxy ou un radical alcoxy en $C_1$-$C_5$,

$Z_1$ est le radical méthyle, et

$Y_1$ est le radical n-hexyle ou n-octyle.

7. Dihydrofuropyridinones selon la revendication 6, caractérisées en ce que, dans la formule (III), $R_5$ et $R_6$ sont chacun le radical méthyle ou éthyle, $X_1$ est le radical éthoxy, $Z_1$ est le radical méthyle et $Y_1$ est le radical n-octyle.

8. Dihydrofuropyridinones selon la revendication 6, caractérisées en ce que, dans la formule (III), -$NR_5R_6$ représente le radical pyrrolidinyle, $X_1$ le radical éthoxy, $Z_1$ le radical méthyle et $Y_1$ le radical n-octyle.

9. Procédé pour la préparation de dihydrofuropyridinones selon la revendication 1, caractérisé en ce qu'on fait réagir un composé de formule (IV)

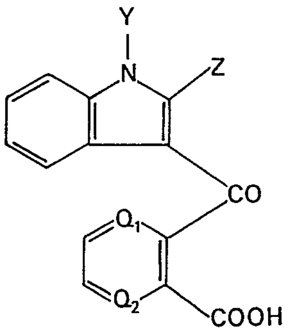

IV

sur un composé de formule (V)

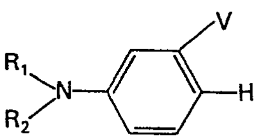

V

dans lesquelles $Q_1$, $Q_2$, Y, Z, $R_1$ et $R_2$ ont les significations données dans la revendication 1, et V a la signification donnée pour X dans la revendication 1 ou est un radical hydroxy, et qu'on alkyle ou aralkyle encore le produit de réaction avec un composé approprié quand V est le radical hydroxy.

10. Procédé pour la préparation de dihydrofuropyridinones selon la revendication 1, caractérisé en ce qu'on fait réagir un composé de formule (VI)

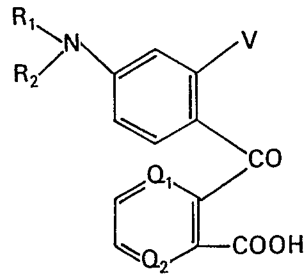

VI

sur un composé de l'indole de formule (VII)

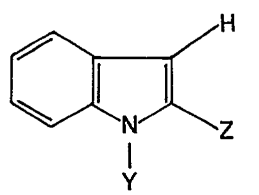

VII

dans lesquelles $Q_1$, $Q_2$, $R_1$, $R_2$, Y et Z ont les significations données dans la revendication 1 et V a la signification donnée pour X dans la revendication 1 ou est le radical hydroxy, et qu'on alkyle ou aralkyle encore le produit de réaction quand V est le radical hydroxy.

11. Utilisation d'un composé de dihydrofuropyridinones ayant la formule donnée dans l'une des revendications 1 à 8, en tant que substance chromogène dans un matériau de'enregistrement sensible à la pression ou sensible à la chaleur.

12. Matériau d'enregistrement sensible à la pression ou à la chaleur, caractérisé en ce qu'il contient en tant que substance chromogène dans son système de réactifs de couleur au moins un composé de dihydrofuropyridinones, ayant une formule selon l'une des revendications 1 à 8.